# EUROPEAN PATENT APPLICATION

(11) **EP 4 681 732 A1**
(43) Date of publication of application: **21.01.2026**
(21) Application number: 24775265.2
(22) Date of filing: 15.03.2024
(51) Int. Cl.: A61K 39/395, A61K 47/20, A61K 47/26, A61K 47/22, A61K 9/00, C07K 16/24, A61K 39/00

(54) **STABLE PHARMACEUTICAL FORMULATION AND PROCESS FOR MANUFACTURING DRUG PRODUCT**

(30) Priority: 17.03.2023 KR 20230035180
(71) Applicant: Celltrion, Inc., Incheon 22014 (KR)
(72) Inventor: LEE, Jae Bin, Incheon 22014 (KR); KIM, Kwang Woo, Incheon 22014 (KR); KIM, Su Jung, Incheon 22014 (KR); SHIN, Yeon Kyeong, Incheon 22014 (KR); OH, Jun Seok, Incheon 22014 (KR); HAN, Won Yong, Incheon 22014 (KR); KIM, Su Ha, Incheon 22014 (KR); CHUNG, Sung Eun, Incheon 22014 (KR)
(74) Representative: Patentanwälte Olbricht Buchhold Keulertz
(86) International application number: PCT/KR2024/095560
(87) International publication number: WO 2024/196220

(57) **Abstract**

A stable pharmaceutical formulation according to the present invention comprises: (A) an antibody or antigen-binding fragment thereof that binds to interleukin-17A (IL-17A); (B) a surfactant; (C) a stabilizer; (D) a buffer; and (E) an antioxidant. The present invention also comprises a pharmaceutical formulation prepared by a step of filling a container with nitrogen and a venting and nitrogen filling step when a rubber stopper is inserted into a pre-filled syringe, in manufacturing a drug product. The stable pharmaceutical formulation according to the present invention has low viscosity even when the formulation comprises an antibody, particularly an antibody in a large amount, has excellent long-term storage stability on the basis of excellent stability under accelerated and harsh conditions, and can be administered intravenously or subcutaneously.

## Description

### Technical Field

The present invention relates to a pharmaceutical formulation capable of stably preserving a drug capable of inhibiting interleukin-17A (IL-17A) and a process for manufacturing a drug product including the same.

### Background Art

Interleukin-17A (IL-17A) is secreted from immune cells, including Th17 and CD8 cells, and promotes the secretion of pro-inflammatory cytokines such as G-SCF, IL-6, and IL-8, which induce immunopathology and neutrophil recruitment. IL-17A can be the cause of immune diseases such as psoriasis, psoriatic arthritis, and ankylosing spondylitis, and a drug containing an antibody that selectively binds to IL-17A can suppress the worsening of the disease by neutralizing IL-17A.

Secukinumab (AIN457) is a high-affinity fully human monoclonal anti-human antibody that inhibits IL-17A activity, which has emerged as a treatment for patients with the above-described immune diseases. It is also published in WO 2006/013107 (PCT/EP2005/008470). Secukinumab (AIN457) was approved by the US FDA in January 2015 for use in the treatment of moderate-to-severe plaque psoriasis, and in January 2016 for use in the treatment of psoriatic arthritis and ankylosing spondylitis. In Korea, it was approved for use in the treatment of plaque psoriasis in September 2015, and for use in the treatment of psoriatic arthritis and ankylosing spondylitis in February 2016.

International Patent Application PCT/IB2015/059836 provides a liquid composition of secukinumab disposed within a container having a headspace including less than about 12% oxygen while maintaining a methionine content of about 20 mM or less. However, PCT/IB2015/059836 did not reflect, in the formulation composition, the concentration range of methionine and the optimization of process technology to achieve the optimal effect in preventing tri-oxidation of LC Cys97 of the antibody. Therefore, those skilled in the art are faced with the technical challenge of producing a commercially viable aqueous biopharmaceutical composition that has optimal anti-oxidation properties while maintaining a balance between myriad requirements (turbidity, RP-HPLC main peak %, SEC-HPLC main peak %, etc.).

Despite the technical challenges outlined above, the inventors have successfully developed novel and beneficial ready-to-use drug products and liquid pharmaceutical compositions of IL-17A antibodies and antigen-binding fragments disclosed herein, e.g., secukinumab.

### DISCLOSURE

### Technical Problem

In the present invention, it has been confirmed that the content of methionine used in the prior art does not sufficiently inhibit the oxidation of pharmaceutical ingredients, and to overcome this, the content of methionine was adjusted to an appropriate level.

In addition, in order to suppress oxidation of pharmaceutical ingredients along with the use of methionine, an antioxidant, a process of filling a container with nitrogen gas during the manufacturing of a drug product including a pharmaceutical formulation and a venting and nitrogen filling process during insertion of a stopper into a pre-filled syringe were added.

The present invention provides a stable pharmaceutical formulation containing: (A) about 150 mg/ml secukinumab; (B) about 30 to 50 mM methionine; (C) about 0.02% (w/v) surfactant; (D) about 170 to 200 mM stabilizer which is a sugar, a sugar alcohol, or a mixture thereof; and (E) about 20 mM histidine buffer.

The present invention also provides a stable pharmaceutical formulation containing: (A) about 150 mg/ml secukinumab; (B) about 30 to 50 mM methionine; (C) about 0.02% (w/v) surfactant; (D) about 170 to 200 mM stabilizer which is a sugar, a sugar alcohol, or a mixture thereof; and (E) about 20 mM histidine buffer, the formulation being prepared through a process of filling a container with nitrogen during the manufacturing of a drug product.

The present invention also provides a stable pharmaceutical formulation containing: (A) about 150 mg/ml secukinumab; (B) about 30 to 50 mM methionine; (C) about 0.02% (w/v) surfactant; (D) about 170 to 200 mM stabilizer which is a sugar, a sugar alcohol, or a mixture thereof; and (E) about 20 mM histidine buffer, the formulation being prepared through a process of filling a container with nitrogen during the manufacturing of a drug product and a venting and nitrogen filling process during insertion of a stopper into a pre-filled syringe.

A stable pharmaceutical formulation according to the present invention may be a stable pharmaceutical formulation having: a. an oxidation rate ≤ 0.7% for a 30 mM methionine-containing formulation prepared through a nitrogen filling process, when measuring the oxidation rate of light-chain Cys97 after 4 weeks of storage under conditions of 40±2°C temperature and 75±5% relative humidity; and/or b. an oxidation rate ≤ 0.5% for a 30 mM methionine-containing formulation prepared through a nitrogen filling process, when measuring the oxidation rate of light-chain Cys97 after 72 hours of storage under conditions of 1,000 lux light illumination, 25±2°C temperature and 60±5% relative humidity; and/or c. an oxidation rate ≤ 0.7% for a 30 mM methionine-containing formulation prepared through a nitrogen filling process, when measuring the oxidation rate of light-chain Cys97 after 4 hours of storage under conditions of agitation at 3,000 RPM.

In one embodiment of the present invention, the pharmaceutical formulation may be a liquid composition, without being limited thereto.

Antibodies that bind to interleukin-17A (IL-17A) are unstable proteins, and are susceptible to physical or chemical changes due to heat stress, light stress, oxidative stress, or the like. Thus, stability and/or activity may be impaired after the product has been stored for a long period of time.

In particular, secukinumab has a cysteine residue that does not form a disulfide bond in the complementarity determining region (CDR) of the antibody, and if the cysteine residue is oxidized, the stability and/or activity of the antibody may be impaired. Thus, inhibition of the oxidation is necessary.

A problem to be solved by the present invention is to provide a stable pharmaceutical formulation containing an antibody that binds to interleukin-17A (IL-17A).

Another problem to be solved by the present invention is to provide a process for manufacturing a drug product capable of stably storing the pharmaceutical formulation.

Another problem to be solved by the present invention is to provide a vial filled with the pharmaceutical formulation.

Another problem to be solved by the present invention is to provide a cartridge filled with the pharmaceutical formulation.

Another problem to be solved by the present invention is to provide a pre-filled syringe filled with the pharmaceutical formulation.

Another problem to be solved by the present invention is to provide an auto-injector having the pre-filled syringe included therein.

### Technical Solution

The present inventors have conducted studies to overcome the above-described problems, and as a result, have developed a stable formulation containing an antibody that binds to interleukin-17A (IL-17A).

The present invention provides a formulation containing methionine, an antioxidant, at a concentration of 30 to 50 mM, and a pharmaceutical formulation prepared through a process of filling a container with nitrogen during the manufacturing of a drug product to inhibit additional oxidation. The liquid composition is not reconstituted from a lyophilizate, but rather may be a ready-to-use liquid composition and may broadly include at least one of the disclosed IL-17 antibodies or antigen-binding fragments thereof, a buffer, a surfactant, methionine, and a stabilizer, as well as subcombinations thereof. The present inventors have found that the combined use of a high content of methionine and technology for filling a container with nitrogen significantly contributes to the stability of the liquid pharmaceutical product, and in particular, effectively inhibits oxidation of the IL-17A antibody (e.g., secukinumab).

Disclosed herein is a drug product including a liquid pharmaceutical composition having a pH of about 5.2 to about 6.2 disposed within a container, the liquid pharmaceutical composition being prepared through a process of filling the container with nitrogen during the manufacturing of the drug product to inhibit additional oxidation and containing about 150 mg/ml secukinumab and about 30 to about 50 mM methionine, wherein the composition is not reconstituted from a lyophilizate.

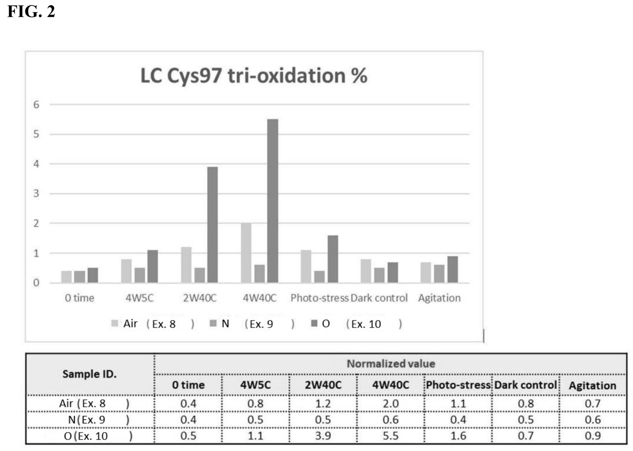

These liquid compositions have excellent properties, for example:
an oxidation rate ≤ 0.7% for a 30 mM methionine-containing formulation prepared through a nitrogen filling process, when measuring the oxidation rate of light-chain Cys97 after 4 weeks of storage under conditions of 40±2°C temperature and 75±5% relative humidity;
an oxidation rate ≤ 0.5% for a 30 mM methionine-containing formulation prepared through a nitrogen filling process, when measuring the oxidation rate of light-chain Cys97 after 72 hours of storage under conditions of 1,000 lux light illumination, 25±2°C temperature and 60±5% relative humidity; and
an oxidation rate ≤ 0.7% for a 30 mM methionine-containing formulation prepared through a nitrogen filling process, when measuring the oxidation rate of light-chain Cys97 after 4 hours of storage under conditions of agitation at 3,000 RPM (FIG. 2).

Additionally, it was confirmed that, when a venting and nitrogen filling process was applied during insertion of a stopper into a pre-filled syringe, it enhanced the effect of inhibiting oxidation of the IL-17A antibody (e.g., secukinumab).

The present invention is also directed to the use of these drug products and stable liquid compositions for the treatment of various IL-17-mediated disorders (e.g., autoimmune disorders, such as psoriasis, ankylosing spondylitis, and psoriatic arthritis) and to kits containing these drug products and stable liquid compositions. Additional compositions, products, methods, regimens, uses, and kits are provided in the following description and appended claims. Further features, advantages and aspects of the present disclosure will become apparent to those skilled in the art from the following description and appended claims.

### (A) Antibody or Antigen-Binding Fragment Thereof

In the present invention, the term "antibody" refers to an immunoglobulin molecule consisting of four polypeptide chains in which two heavy chains and two light-chains are linked to each other by disulfide bonds, and may include naturally occurring antibodies having other changed structures, such as camelid antibodies.

In one embodiment of the present invention, the antibody or antigen-binding fragment thereof according to the present invention may be a polyclonal antibody, a monoclonal antibody, a recombinant antibody, a single-chain antibody, a hybrid antibody, a chimeric antibody, a humanized antibody, a fully human antibody, or a fragment thereof (antigen-binding fragment), preferably a monoclonal antibody or a fragment thereof.

In the present invention, the antibody or antigen-binding fragment thereof according to the present invention may be a fully human monoclonal antibody or a fragment thereof, most preferably a fully human immunoglobulin G (IgG) monoclonal antibody, and may be produced by a known method.

In the present disclosure, the antigen-binding fragment includes any naturally occurring, enzymatically obtainable, synthetic or genetically engineered polypeptide or glycoprotein that specifically binds to an antigen to form a complex, examples of which include nanobodies and the like.

In the present invention, the antibody (A) may be secukinumab, ixekizumab, bimekizumab, or a mixture thereof. Preferably, the antibody may be secukinumab, ixekizumab, bimekizumab, or a mixture thereof. Most preferably, the antibody may be secukinumab.

In the present invention, the "secukinumab" may be secukinumab described in International Publication No. WO2016-103153, the original drug substance as well known in the art, or a biosimilar thereof.

In one embodiment of the present invention, the secukinumab antibody may comprise: a light-chain variable region comprising a CDR1 domain comprising the amino acid sequence of SEQ ID NO: 1, a CDR2 domain comprising the amino acid sequence of SEQ ID NO: 2, and a CDR3 domain comprising the amino acid sequence of SEQ ID NO: 3; and a heavy chain variable region comprising a CDR1 domain comprising the amino acid sequence of SEQ ID NO: 4, a CDR2 domain comprising the amino acid sequence of SEQ ID NO: 5, and a CDR3 domain comprising the amino acid sequence of SEQ ID NO: 6.

In one embodiment of the present invention, the secukinumab antibody may comprise: a light-chain variable region comprising the amino acid sequence of SEQ ID NO: 7; and a heavy chain variable region comprising the amino acid sequence of SEQ ID NO: 8.

In one embodiment of the present invention, the secukinumab antibody may comprise: a light-chain comprising the amino acid sequence of SEQ ID NO: 9; and a heavy chain comprising the amino acid sequence of SEQ ID NO: 10.

In the present invention, the "ixekizumab" may be an interleukin-17A (IL-17A)-binding antibody, also known as Taltz^{®}, or a biosimilar thereof.

In the present invention, the "bimekizumab" may be an interleukin-17A (IL-17A)-binding antibody, also known as Bimzelx^{®}, or a biosimilar thereof.

In one embodiment of the present invention, the concentration of the antibody or antigen-binding fragment thereof (A) may be 1 to 300 mg/mL, preferably 150 mg/mL. When the concentration of the antibody or antigen-binding fragment thereof is within the above-described range, the degree of freedom of administration dose and administration cycle may be increased, and long-term stability is excellent.

A high-concentration protein formulation (HCPF) refers to a formulation containing mainly a monoclonal antibody (mAb) drug at a high concentration, wherein the concentration of mAb is typically in the range of 50 to 150 mg/mL. High-concentration protein formulations have stability and colloidal properties different from those of formulations at a low protein concentration (e.g., 10 mg/mL) due to the complexity of the molecules themselves and various intermolecular interactions. As the protein concentration increases, aggregation and degradation are promoted, making it difficult to develop a stable pharmaceutical formulation. In addition, reversible self-association occurs, which affects an increase in viscosity, etc., and causes difficulties in developing a stable formulation and manufacturing a drug product.

### (B) Surfactant

In the present invention, the term "surfactant" refers to a substance which may be used to significantly increase the water solubility of a hydrophobic or oily substance or increase the miscibility of two substances having different hydrophobic properties.

In the present invention, the surfactant according to the present invention may be polyoxyethylene sorbitan fatty acid ester (e.g., polysorbate, etc.), polyoxyethylene alkyl ether (e.g., Brij^{®}, etc.), alkylphenylpolyoxyethylene ether (e.g., Triton-X, etc.), a polyoxyethylene-polyoxypropylene copolymer (e.g., poloxamer, Pluronic^{®}, etc.), sodium dodecyl sulfate (SDS), or a mixture thereof, without being limited thereto.

The surfactant may be preferably a polyoxyethylene sorbitan fatty acid ester, a polyoxyethylene-polyoxypropylene copolymer, or a mixture thereof, more preferably a polyoxyethylene sorbitan fatty acid ester.

In the present invention, the surfactant may be polysorbate, poloxamer, or a mixture thereof, preferably polysorbate.

In the present invention, the surfactant may be polysorbate 20, polysorbate 40, polysorbate 60, polysorbate 80, or a mixture thereof, preferably polysorbate 80.

In one embodiment of the present invention, the concentration of the surfactant may be freely adjusted within a range that does not adversely affect the stability and viscosity of the stable pharmaceutical formulation according to the present invention.

In one embodiment of the present invention, the concentration of the surfactant (B) may be 0.001 to 1% (w/v), preferably 0.02 (w/v). When the concentration of the surfactant is within the above-mentioned range, it exhibits excellent long-term stability and low viscosity.

### (C) Stabilizer

In the present invention, the term "stabilizer" refers to a substance that is physiologically acceptable and imparts stability to the formulation.

In one embodiment of the present invention, the stabilizer (C) may be a sugar, a sugar alcohol, or a mixture thereof.

In one embodiment of the present invention, the sugar or the sugar alcohol may be sucrose, trehalose, sorbitol, glucose, fructose, galactose, xylose, maltose, lactose, xylitol, mannitol, D-maltitol, inositol, lactitol, or isomalt, preferably trehalose, without being limited thereto.

In the present invention, the concentration of the stabilizer may be freely adjusted within a range that does not substantially adversely affect the stability and viscosity of the pharmaceutical formulation according to the present invention.

In one embodiment of the present invention, the concentration of the stabilizer (C) may be 100 to 250 mM, preferably 170 to 200 mM, more preferably 170 mM.

In the present invention, when the concentration of the stabilizer according to the present disclosure is within the range described in the present invention, long-term stability and low viscosity are excellent.

### (D) Buffer

In the present invention, the term "buffer" refers to a substance that minimizes the change in pH caused by acid or alkali.

In one embodiment of the present invention, the buffer (D) may be histidine or a salt thereof, acetic acid or a salt thereof, phosphoric acid or a salt thereof, citric acid or a salt thereof, succinic acid or a salt thereof, glutamic acid or a salt thereof, 2-(N-morpholino) ethanesulfonic acid (MES) buffer, tromethamine (Tris) or a salt thereof, or the like, without being limited thereto. The buffer may be histidine or a salt thereof, acetic acid or a salt thereof, phosphoric acid or a salt thereof, citric acid or a salt thereof, succinic acid or a salt thereof, or a mixture thereof.

In the present invention, the buffer may be preferably histidine or a salt thereof, or a mixture thereof.

For example, the histidine salt according to the present invention may be histidine chloride, histidine acetate, histidine phosphate, histidine sulfate, or the like, without being limited thereto.

For example, the salt of acetic acid according to the present invention may be sodium acetate, zinc acetate, aluminum acetate, ammonium acetate, potassium acetate, or the like, without being limited thereto.

For example, the salt of phosphoric acid according to the present invention may be potassium phosphate, sodium phosphate, ammonium phosphate, calcium phosphate, magnesium phosphate, or the like, without being limited thereto.

For example, the salt of citric acid according to the present invention may be sodium citrate, calcium citrate, potassium citrate, or the like, without being limited thereto.

For example, the salt of succinic acid according to the present invention may be sodium succinate, calcium succinate, potassium succinate, sodium sulfosuccinate, potassium sulfosuccinate, calcium sulfosuccinate, or the like, without being limited thereto.

For example, the salt of glutamic acid according to the present invention may be sodium glutamate, potassium glutamate, ammonium glutamate, or the like, without being limited thereto.

For example, the salt of 2-(N-morpholino) ethanesulfonic acid (MES) salt according to the present invention may be MES chloride, MES sodium, or the like, without being limited thereto.

For example, the salt of Tris according to the present invention may be Tris hydrogen chloride, Tris acetate, Tris borate, or the like, without being limited thereto.

In the present invention, the content of the buffer may be freely adjusted within a range that does not substantially adversely affect the stability and viscosity of the pharmaceutical formulation according to the present invention.

In one embodiment of the present invention, the content of the buffer (D) may be 0.1 to 50 mM, preferably 20 mM.

### (E) Antioxidant

In the present invention, the term "antioxidant" refers to a substance that inhibits the oxidation-induced degradation of the activity of the antibody and the stability of the pharmaceutical formulation.

In one embodiment of the present invention, the antioxidant (E) may be methionine, ascorbic acid or a salt thereof, citric acid or a salt thereof, taurine, or the like, without being limited thereto.

For example, the salt of citric acid according to the present invention may be sodium citrate, calcium citrate, potassium citrate, or the like, without being limited thereto.

For example, the salt of ascorbic acid according to the present invention may be sodium ascorbate, calcium ascorbate, potassium ascorbate, magnesium ascorbate, or the like, without being limited thereto.

In the present invention, the antioxidant may preferably be methionine.

In one embodiment of the present invention, the concentration of the antioxidant (E) may be 0 to 100 mM, preferably 30 to 50 mM, more preferably 30 mM.

### (F) pH

In the present invention, the pH of the stable pharmaceutical formulation according to the present invention may be adjusted within a range that optimizes the therapeutic efficacy by using a buffer.

In the present invention, the pH may be 5 to 7, preferably 5.8.

In one embodiment of the present invention, when a buffer is contained in a predetermined amount, the pharmaceutical formulation may exhibit a pH within the above-described range without a separate pH adjusting agent. In one embodiment of the present invention, the pharmaceutical formulation may further contain a separate pH adjusting agent (acid or base) within a range that does not impair the stability of the antibody.

In the present invention, the term "pH adjusting agent" refers to a substance that is used to adjust the pH of the stable pharmaceutical formulation.

In one embodiment of the present invention, the pharmaceutical formulation may further contain a separate pH adjusting agent (acid or base) within a range that does not impair the stability of the pharmaceutical formulation.

In the present invention, the content of the pH adjusting agent may be freely adjusted within a range that does not substantially adversely affect the stability and viscosity of the pharmaceutical formulation according to the present invention.

In one embodiment of the present invention, the pH adjusting agent (E) may be hydrochloric acid, acetic acid, phosphoric acid, sodium hydroxide, sodium hydrogen carbonate, or a mixture thereof.

### (G) Other Ingredients

In the present invention, the stable pharmaceutical formulation according to the present invention may not include a preservative. For example, the preservative may be octadecyldimethylbenzyl ammonium chloride, hexamethonium chloride, benzalkonium chloride, benzethonium chloride, phenol, butyl alcohol, benzyl alcohol, alkyl paraben, catechol, resorcinol, cyclohexanol, 3-pentanol, m-cresol, or the like. If the preservative is included, it may have a negative effect on improving stability.

As used herein, the term "not including" or "does not include" means that any component is not included at all. In addition, the term means that any component is not substantially included, that is, any component is included in a range that does not affect the activity of the antibody, the stability or viscosity of the pharmaceutical formulation. For example, the term means that any component is included in an amount of 1% (w/v) or less, 1 mM or less, 1 mg/ml or less, 1 ppm (w/v) or less, or 1 ppb (w/v) or less based on the total weight of the pharmaceutical formulation.

In the present invention, the stable pharmaceutical formulation of the present invention may further contain an additive known in the art within a range that does not substantially adversely affect the stability and viscosity of the formulation. The additive may be, for example, an aqueous carrier, or a mixture thereof. In the present invention, the aqueous carrier may be a carrier useful for the preparation of a pharmaceutical formulation, which is safe and non-toxic when administered to humans. Examples of the aqueous carrier include, but are not limited to, sterile water for injection (SWFI), bacteriostatic water for injection (BWFI), sterile saline solution, Ringer's solution, dextrose, and the like.

### (H) "Stable" Pharmaceutical Formulation

In the present invention, the term "stable" or "stabilization" means that during the manufacturing process and/or during storage, the component according to the present invention or a composition or formulation containing the same exhibits its physical stability, chemical stability and/or biological activity. In the present invention, various analytical techniques for measuring stability are readily available in the art.

In the present invention, the physical stability may be evaluated by a method known in the art, for example, it may be evaluated by measuring the sample apparent attenuation of light (absorption or optical density). This measurement of light attenuation is related to the turbidity of the formulation. In addition, the content of a high-molecular-weight component, the content of a low-molecular-weight component, the amount of intact protein, and the like may be measured for physical stability.

In one embodiment of the present invention, the term "stable" pharmaceutical formulation means a pharmaceutical formulation satisfying one or more of the following (H)-1 to (H)-9.

### (H)-1 Turbidity

- A pharmaceutical formulation having an absorbance A₃₅₀ of 0 to 0.3, as measured by a spectrophotometer after storage for 0 days, 10 days, 3 weeks, or 4 weeks at 5±3°C temperature;
- A pharmaceutical formulation having an absorbance A₃₅₀ of 0 to 0.6, as measured by a spectrophotometer after storage for 0 days, 1 week, 2 weeks, 3 weeks, or 4 weeks under conditions of 40±2°C temperature and 75±5% relative humidity;
- A pharmaceutical formulation having an absorbance A₃₅₀ of 0 to 0.6, as measured by a spectrophotometer after storage for 0 days, 5 days, or 10 days under conditions of 50°C temperature;
- A pharmaceutical formulation having an absorbance A₃₅₀ of 0 to 0.6, as measured by a spectrophotometer after storage for 72 hours under conditions of 1,000 Lux light illumination, 25±2°C temperature, and 60±5% relative humidity;
- A pharmaceutical formulation having an absorbance A₃₅₀ of 0 to 0.3, as measured by a spectrophotometer after storage for 4 hours under conditions of agitation at 3,000 RPM;

### (H)-2 RP-HPLC main peak

- A pharmaceutical formulation having a main peak of 83% to 100%, as measured by RP-HPLC after storage for 0 days, 10 days, 3 weeks or 4 weeks at 5±3°C temperature;
- A pharmaceutical formulation having a main peak of 63% to 100%, as measured by RP-HPLC after storage for 0 days, 1 week, 2 weeks, 3 weeks, or 4 weeks under conditions of 40±2°C temperature and 75±5% relative humidity;
- A pharmaceutical formulation having a main peak of 55% to 100%, as measured by RP-HPLC after storage for 0 days, 5 days, 1 week, 10 days, 2 weeks, or 3 weeks under conditions of 50°C temperature;
- A pharmaceutical formulation having a main peak of 82% to 100%, as measured by RP-HPLC after storage for 72 hours under conditions of 1,000 Lux light illumination, 25±2°C temperature, and 60±5% relative humidity;
- A pharmaceutical formulation having a main peak of 84% to 100% as measured by RP-HPLC after storage for 4 hours under conditions of agitation at 3,000 RPM;

### (H)-3 RP-HPLC pre-peak (peak with retention time before main peak)

- A pharmaceutical formulation having a pre-peak of 0 to 5%, as measured by RP-HPLC after storage for 0 days, 10 days, 3 weeks, or 4 weeks at 5±3°C temperature;
- A pharmaceutical formulation having a pre-peak of 0 to 30%, as measured by RP-HPLC after storage for 0 days, 1 week, 2 weeks, 3 weeks, or 4 weeks under conditions of 40±2°C temperature and 75±5% relative humidity;
- A pharmaceutical formulation having a pre-peak of 0 to 37%, as measured by RP-HPLC after storage for 0 days, 5 days, 1 week, 10 days, 2 weeks, or 3 weeks under conditions of 50°C temperature;
- A pharmaceutical formulation having a pre-peak of 0 to 7%, as measured by RP-HPLC after storage for 72 hours under conditions of 1,000 lux light illumination, 25±2°C temperature, and 60±5% relative humidity;
- A pharmaceutical formulation having a pre-peak of 0 to 6%, as measured by RP-HPLC after storage for 4 hours under conditions of agitation at 3,000 RPM;

### (H)-4 RP-HPLC post-peak (peak with retention time after main peak)

- A pharmaceutical formulation having a post-peak of 0 to 13%, as measured by RP-HPLC after storage for 0 days, 10 days, 3 weeks, or 4 weeks at 5±3°C temperature;
- A pharmaceutical formulation having a post-peak of 0 to 12%, as measured by RP-HPLC after storage for 0 days, 1 week, 2 weeks, 3 weeks, or 4 weeks under conditions of 40±2°C temperature and 75±5% relative humidity;
- A pharmaceutical formulation having a post-peak of 0 to 11%, as measured by RP-HPLC after storage for 0 days, 5 days, 1 week, 10 days, 2 weeks, or 3 weeks under conditions of 50°C temperature;
- A pharmaceutical formulation having a post-peak of 0 to 12%, as measured by RP-HPLC after storage for 72 hours under conditions of 1,000 lux light irradiation, 25±2°C temperature, and 60±5% relative humidity;
- A pharmaceutical formulation having a post-peak of 0 to 12%, as measured by RP-HPLC after storage for 4 hours under conditions of agitation at 3,000 RPM;

### (H)-5 SEC-HPLC monomer (main peak)

- A pharmaceutical formulation having a monomer component of 94% to 100%, as measured by SEC-HPLC after storage for 0 days, 10 days, 3 weeks or 4 weeks at 5±3°C temperature;
- A pharmaceutical formulation having a monomer component of 85% to 100%, as measured by SEC-HPLC after storage for 0 days, 1 week, 2 weeks, 3 weeks, or 4 weeks under conditions of 40±2°C temperature and 75±5% relative humidity;
- A pharmaceutical formulation having a monomer component of 77% to 100%, as measured by SEC-HPLC after storage for 0 days, 5 days, 1 week, 10 days, 2 weeks, or 3 weeks under 50°C temperature;
- A pharmaceutical formulation having a monomer component of 94% to 100%, as measured by SEC-HPLC after storage for 72 hours under conditions of 1,000 lux light illumination, 25±2°C temperature, and 60±5% relative humidity;
- A pharmaceutical formulation having a monomer component of 94% to 100%, as measured by SEC-HPLC after storage for 4 hours under conditions of agitation at 3,000 RPM;

### (H)-6 SEC-HPLC high-molecular-weight component (peak with retention time before main peak)

- A pharmaceutical formulation having a high-molecular-weight component of 0 to 3%, as measured by SEC-HPLC after storage for 0 days, 10 days, 3 weeks, or 4 weeks at 5±3°C temperature;
- A pharmaceutical formulation having a high-molecular-weight component of 0 to 5%, as measured by SEC-HPLC after storage for 0 days, 1 week, 2 weeks, 3 weeks, or 4 weeks under conditions of 40±2°C temperature and 75±5% relative humidity;
- A pharmaceutical formulation having a high-molecular-weight component of 0 to 7%, as measured by SEC-HPLC after storage for 0 days, 5 days, 1 week, 10 days, 2 weeks, or 3 weeks under conditions of 50°C temperature;
- A pharmaceutical formulation having a high-molecular-weight component of 0 to 3%, as measured by SEC-HPLC after storage for 72 hours under conditions of 1,000 lux light illumination, 25±2°C temperature, and 60±5% relative humidity;
- A pharmaceutical formulation having a high-molecular-weight component of 0 to 3%, as measured by SEC-HPLC after storage for 4 hours under conditions of agitation at 3,000 RPM;

### (H)-7 SEC-HPLC low-molecular-weight component (peak with retention time after main peak)

- A pharmaceutical formulation having a low-molecular-weight component of 0 to 3%, as measured by SEC-HPLC after storage for 0 days, 10 days, 3 weeks, or 4 weeks at 5±3°C temperature;
- A pharmaceutical formulation having a low-molecular-weight component of 0 to 10%, as measured by SEC-HPLC after storage for 0 days, 1 week, 2 weeks, 3 weeks, or 4 weeks under conditions of 40±2°C temperature and 75±5% relative humidity;
- A pharmaceutical formulation having a low-molecular-weight component of 0 to 16%, as measured by SEC-HPLC after storage for 0 days, 5 days, 1 week, 10 days, 2 weeks, or 3 weeks under conditions of 50°C temperature;
- A pharmaceutical formulation having a low-molecular-weight component of 0 to 3%, as measured by SEC-HPLC after storage for 72 hours under conditions of 1,000 lux light illumination, 25±2°C temperature, and 60±5% relative humidity;
- A pharmaceutical formulation having a low-molecular-weight component of 0 to 4%, as measured by SEC-HPLC after storage for 4 hours under conditions of agitation at 3,000 RPM;

### (H)-8 Methionine oxidation rate

- A pharmaceutical formulation having an oxidation rate of heavy-chain Met262 of 0 to 2%, as measured by LC-MS after storage for 0 days, 10 days, 3 weeks, or 4 weeks at 5±3°C temperature;
- A pharmaceutical formulation having an oxidation rate of heavy-chain Met262 of 0 to 5%, as measured by LC-MS after storage for 0 days, 1 week, 2 weeks, 3 weeks, or 4 weeks under conditions of 40±2°C temperature and 75±5% relative humidity;
- A pharmaceutical formulation having an oxidation rate of heavy-chain Met262 of 0 to 6%, as measured by LC-MS after storage for 0 days, 5 days, 1 week, 10 days, 2 weeks, or 3 weeks under conditions of 50°C temperature;
- A pharmaceutical formulation having an oxidation rate of heavy-chain Met262 of 0 to 3%, as measured by LC-MS after storage for 72 hours under conditions of 1,000 lux light illumination, 25±2°C temperature, and 60±5% relative humidity;
- A pharmaceutical formulation having an oxidation rate of heavy-chain Met262 of 0 to 2%, as measured by LC-MS after storage for 4 hours under conditions of agitation at 3,000 RPM;

### (H)-9 Cysteine oxidation rate

- A pharmaceutical formulation having an oxidation rate of light-chain Cys97 of 0 to 3%, as measured by LC-MS after storage for 0 days, 10 days, 3 weeks, or 4 weeks at 5±3°C temperature;
- A pharmaceutical formulation having an oxidation rate of light-chain Cys97 of 0 to 10%, as measured by LC-MS after storage for 0 days, 1 week, 2 weeks, 3 weeks, or 4 weeks under conditions of 40±2°C temperature and 75±5% relative humidity;
- A pharmaceutical formulation having an oxidation rate of light-chain Cys97 of 0 to 7%, as measured by LC-MS after storage for 0 days, 5 days, 1 week, 10 days, 2 weeks, or 3 weeks under conditions of 50°C temperature;
- A pharmaceutical formulation having an oxidation rate of light-chain Cys97 of 0 to 2%, as measured by LC-MS after storage for 72 hours under conditions of 1,000 lux light illumination, 25±2°C temperature, and 60±5% relative humidity;
- A pharmaceutical formulation having an oxidation rate of light-chain Cys97 of 0 to 2%, as measured by LC-MS after storage for 4 hours under conditions of agitation at 3,000 RPM.

### [Product]

In one embodiment of the present invention, the present invention provides a product including: the stable pharmaceutical formulation according to the present invention; and a container accommodating the stable pharmaceutical preparation in a sealed state.

The stable pharmaceutical formulation is as described above herein.

In the present invention, the container may be formed of a material such as glass, a polymer (e.g., plastic), a metal, or the like, without being limited thereto.

For example, the container according to the present invention may be a bottle, a vial, a cartridge, a syringe (e.g., a pre-filled syringe, etc.), an auto-injector, or a tube, preferably a vial made of glass or a polymer, or a pre-filled syringe made of glass or a polymer, without being limited thereto.

In one embodiment of the present invention, the present invention provides a vial filled with the pharmaceutical formulation according to the present invention, a cartridge filled with the pharmaceutical formulation, a pre-filled syringe filled with the pharmaceutical formulation, or an auto-injector having the pre-filled syringe included therein.

Specific product types of the vial, cartridge, pre-filled syringe, auto-injector, etc., and the method of filling the vial, cartridge, pre-filled syringe, auto-injector, etc. with the stable pharmaceutical formulation may be easily obtained or implemented by those of ordinary skill in the art to which the present invention pertains. For example, U.S. Pat. Nos. 4,861,335, 6,331,174 and the like disclose specific product types and filling methods of pre-filled syringes. For example, U.S. Pat. Nos. 5,085,642, 5,681,291 and the like disclose specific product types and assembly methods of auto-injectors. As the vial, cartridge, pre-filled syringe, auto-injector, etc., a commercially available product may be used without change, or a separately custom-made product may be used in consideration of the physical properties, administration site, dose and the like of the stable pharmaceutical formulation.

In the present invention, the container may be a single-dose container.

In the present invention, the product according to the present invention may further include instructions providing a method of using the stable pharmaceutical formulation, a method of storing the stable pharmaceutical formulation, or both. The instructions may include a treatment method for diseases in which the activity of interleukin-17A (IL-17A) is detrimental, the route of administration, dosage, or the timing of administration.

In the present invention, the product may include other tools necessary from a commercial and user point of view, for example, a needle, a syringe, and the like.

### [Method for Preparing Stable Pharmaceutical Formulation]

The stable pharmaceutical formulation of the present invention may be prepared by a known method, and is not limited to a specific preparation method. For example, the pharmaceutical formulation may be prepared by adjusting the pH by adding a buffer to a solution containing a surfactant and a stabilizer, and then adding an antibody to the mixed solution. As another example, the pharmaceutical formulation may be prepared by preparing a solution including an antibody, a buffer and a stabilizer in the final step of the purification process, and then adding a surfactant to the solution.

The air within the container which is filled with the stable pharmaceutical formulation of the present invention may be replaced with an inert gas. For example, the container may be filled with nitrogen through nitrogen purge, thereby enhancing the oxidation stability of the pharmaceutical formulation.

The stable pharmaceutical formulation of the present invention may be filled into a pre-filled syringe, and the insertion of a stopper into the pre-filled syringe may be performed through a venting process. The venting process may be performed in the following order: inserting a ventilation tube into the pre-filled syringe, inserting a stopper into the ventilation tube using an insertion rod, removing the insertion rod, and removing the ventilation tube.

The process of inserting the stopper into the pre-filled syringe using the venting process according to the present invention may be performed together with nitrogen filling. For example, the process of inserting the stopper using the venting process may be performed immediately after filling the pre-filled syringe with nitrogen.

The stable pharmaceutical formulation of the present invention may be filled into a pre-filled syringe, and the insertion of a stopper into the pre-filled syringe may be performed through a vacuum process. The vacuum process may be performed in the following order: attaching a vacuum tube to the pre-filled syringe, reducing the pressure within the pre-filled syringe, placing a stopper on the top of the pre-filled syringe plunger, releasing the vacuum, and moving the stopper.

### [Method of Using Stable Pharmaceutical Formulation]

In the present invention, the stable pharmaceutical formulation according to the present invention may exhibit a therapeutic effect on a disease associated with interleukin-17A (IL-17A).

In the present invention, the stable pharmaceutical formulation according to the present invention may be used for intravenous (IV) administration or subcutaneous (SC) administration.

In one embodiment of the present invention, the stable pharmaceutical formulation may not be subjected to a reconstitution step prior to use.

The concentrations of other components as well as the antibody in the pharmaceutical formulation are as described above, and the total volume of the pharmaceutical formulation according to the present invention may be 0.5 to 2.0 mL.

The dosage and timing of administration of the pharmaceutical formulation may vary depending on the type of disease, the severity and course of the disease, the patient's health and response to treatment, and the judgment of the treating doctor, and are not limited to a specific dosage and timing of administration.

### [Treatment Method and Stabilization Method]

In one embodiment of the present invention, the present invention provides a method for treating a disease in which the activity of interleukin-17A is detrimental, including administering, to a patient having the disease in which the activity of interleukin-17A is detrimental, a stable pharmaceutical formulation containing (A) an antibody or antigen-binding fragment thereof that binds to an interleukin-17A receptor, (B) a surfactant, (C) a stabilizer, (D) a buffer, and (E) an antioxidant.

In another embodiment of the present invention, the present invention provides a method for stabilizing an antibody in a pharmaceutical formulation, including preparing a stable pharmaceutical formulation containing (A) an antibody or antigen-binding fragment thereof that binds to interleukin-17, (B) a surfactant, (C) a stabilizer, (D) a buffer, and (E) an antioxidant.

Specifically, the present invention provides a stable pharmaceutical formulation containing: (A) about 150 mg/ml secukinumab; (B) about 30 to 50 mM methionine; (C) about 0.02% (w/v) surfactant; (D) about 170 to 200 mM stabilizer, which is a sugar, a sugar alcohol, or a mixture thereof; and (E) about 20 mM histidine buffer.

In addition, in another embodiment, the present invention provides a method for stabilizing an antibody in a pharmaceutical formulation, including providing a stable pharmaceutical formulation containing: (A) about 150 mg/ml secukinumab; (B) about 30 to 50 mM methionine; (C) about 0.02% (w/v) surfactant; (D) about 170 to 200 mM stabilizer, which is a sugar, a sugar alcohol, or a mixture thereof; and (E) about 20 mM histidine buffer, wherein the formulation is prepared through a process of filling a container with nitrogen during the manufacturing of a drug product.

In addition, in another embodiment, the present invention provides a method for stabilizing an antibody in a pharmaceutical formulation, including providing a stable pharmaceutical formulation containing: (A) about 150 mg/ml secukinumab; (B) about 30 to 50 mM methionine; (C) about 0.02% (w/v) surfactant; (D) about 170 to 200 mM stabilizer, which is a sugar, a sugar alcohol, or a mixture thereof; and (E) about 20 mM histidine buffer, wherein the formulation is prepared through a process of filling a container with nitrogen during the manufacturing of a drug product and a venting and nitrogen filling process during insertion of a stopper into a pre-filled syringe.

The stable pharmaceutical formulation is as described above herein.

For the treatment method or stabilization method according to the present invention, the above description in the present specification applies.

The above-described individual features described herein may be used in combination, and the fact that the above-described individual features are set forth in different dependent claims of the appended claims does not indicate that they cannot be used in combination.

### Advantageous Effects

The stable pharmaceutical formulation according to the present invention has a low viscosity even when containing an antibody, especially at a high concentration, and has excellent long-term storage stability based on excellent stability under accelerated and stressed conditions, and may be administered intravenously or subcutaneously.

### Brief Description of Drawings

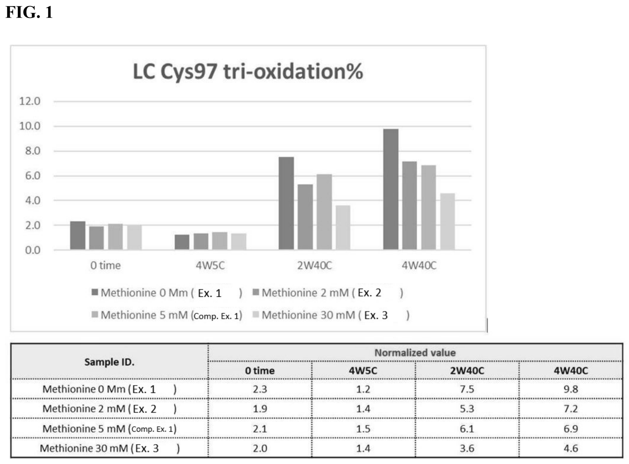

FIGS. 1, 2, and 3 show light-chain Cys97 oxidation rate (%) under each experimental condition.

### Mode for Invention

Hereinafter, the present invention will be described in detail through experimental examples. The following experimental examples are only illustrative of the present invention, and the scope of present invention is not limited by the following experimental examples. The documents referred to in the present invention are incorporated in the specification of the present invention by reference.

With respect to the antibody used in the following experimental examples, secukinumab incubated and purified at the Celltrion Research Institute was used. The physical stability, chemical stability, and biological activity of the pharmaceutical formulations described below were measured using the following methods.

- Turbidity

The absorbance at 350 nm was measured using a UV-Vis spectrophotometer.
- RP-HPLC main peak

Main peak (%) was measured using reverse-phase high-performance liquid chromatography (SEC-HPLC).
- RP-HPLC pre-peak

Pre-peak (%) was measured using reverse-phase high-performance liquid chromatography (size-exclusion high-performance liquid chromatography; SEC-HPLC).
- RP-HPLC post-peak

Post-peak (%) was measured using reverse-phase high-performance liquid chromatography (size-exclusion high-performance liquid chromatography; SEC-HPLC).
- SEC-HPLC main component

Monomer (%) was measured using size-exclusion high-performance liquid chromatography (SEC-HPLC).
- SEC-HPLC high-molecular-weight component

High-molecular-weight component (HMW; %) was measured using size-exclusion high-performance liquid chromatography (SEC-HPLC).
- SEC-HPLC low-molecular-weight component

Low-molecular-weight component (LMW; %) was measured using size-exclusion high-performance liquid chromatography (SEC-HPLC).
- Methionine oxidation rate

The oxidation rate (%) of heavy-chain Met262 was measured by peptide mapping using liquid chromatography-mass spectrometry (LC-MS).
- Cysteine oxidation rate

The oxidation rate (%) of light-chain Cys97 was measured by peptide mapping using liquid chromatography-mass spectrometry (LC-MS).

### Experimental Example 1: Preparation of Pharmaceutical Formulation Containing Antibody That Binds to Interleukin-17A (IL-17A)

Examples 1 to 13 in Table 1 below were prepared by preparing each buffer according to each pH, and then adding a stabilizer and an antioxidant thereto, followed by the addition of an antibody and then a surfactant. Comparative Example 1 and 2 were prepared by applying the same formulation as the originally developed drug product to the same antibody as in the Examples, and were analyzed for comparison with each Example. The specific content of each component is shown in Table 1 below.

**[Table 1]**

| | Antibody | Surfactant | Stabilizer | Buffer | Antioxidant | pH | Container | Stopper insertion process | Filling gas |
|---|---|---|---|---|---|---|---|---|---|
| **Example 1** | 150 mg/mL | Polysorbate 80 0.02% (w/v) | Trehalose 200 mM | Histidine 20 mM | N/A | 5.8 | Vial | N/A | Air |
| **Example 2** | 150 mg/mL | Polysorbate 80 0.02% (w/v) | Trehalose 200 Mm | Histidine 20 mM | Methionine 2 mM | 5.8 | Vial | N/A | Air |
| **Example 3** | 150 mg/mL | Polysorbate 80 0.02% (w/v) | Trehalose 200 mM | Histidine 20 mM | Methionine 30 mM | 5.8 | Vial | N/A | Air |
| **Example 4** | 150 mg/mL | Polysorbate 80 0.02% (w/v) | Trehalose 200 mM | Histidine 20 mM | N/A | 5.8 | Vial | N/A | Air |
| **Example 5** | 150 mg/mL | Polysorbate 80 0.02% (w/v) | Trehalose 200 mM | Histidine 20 mM | Methionine 30 mM | 5.8 | Vial | N/A | Air |
| **Example 6** | 150 mg/mL | Polysorbate 80 0.02% (w/v) | Trehalose 200 mM | Histidine 20 mM | Methionine 40 mM | 5.8 | Vial | N/A | Air |
| **Example 7** | 150 mg/mL | Polysorbate 80 0.02% (w/v) | Trehalose 200 mM | Histidine 20 mM | Methionine 50 mM | 5.8 | Vial | N/A | Air |
| **Example 8** | 150 mg/mL | Polysorbate 80 0.02% (w/v) | Trehalose 170 mM | Histidine 20 mM | Methionine 30 mM | 5.8 | Vial | N/A | Air |
| **Example 9** | 150 mg/mL | Polysorbate 80 0.02% (w/v) | Trehalose 170 mM | Histidine 20 mM | Methionine 30 Mm | 5.8 | Vial | N/A | Nitrogen |
| **Example 10** | 150 mg/mL | Polysorbate 80 0.02% (w/v) | Trehalose 170 mM | Histidine 20 mM | Methionine 30 mM | 5.8 | Vial | N/A | Oxygen |
| **Example 11** | 150 mg/mL | Polysorbate 80 0.02% (w/v) | Trehalose 170 mM | Histidine 20 mM | Methionine 30 mM | 5.8 | Pre-filled syringe | Venting | Air |
| **Example 12** | 150 mg/mL | Polysorbate 80 0.02% (w/v) | Trehalose 170 mM | Histidine 20 mM | Methionine 30 mM | 5.8 | Pre-filled syringe | Vacuum | Air |
| **Example 13** | 150 mg/mL | Polysorbate 80 0.02% (w/v) | Trehalose 170 mM | Histidine 20 mM | Methionine 30 mM | 5.8 | Pre-filled syringe | Venting/nit rogen filling | Nitrogen |
| **Comp. Example 1** | 150 mg/mL | Polysorbate 80 0.02% (w/v) | Trehalose 200 mM | Histidine 20 mM | Methionine 5 mM | 5.8 | Vial | N/A | Air |
| **Comp. Example 2** | 150 mg/mL | Polysorbate 80 0.02% (w/v) | Trehalose 200 mM | Histidine 20 mM | Methionine 5 mM | 5.8 | Vial | N/A | Air |

### Experimental Example 2: First Comparison of Antioxidant

The stability of Examples 1 to 3 prepared according to Experimental Example 1 was measured under conditions of 5+3°C temperature and conditions of 40+2°C 5 temperature and 75±5% relative humidity, and the results are shown in Table 2 below.

**[Table 2]**

| Evaluation Examples and results | Example 1 | Example 2 | Example 3 | Comparative Example 1 | |
|---|---|---|---|---|---|
| Antioxidant | Not containing | Methionine 2 mM | Methionine 30 mM | Methionine 5 mM | |

| Antibody (mg/ml) | | 150 | 150 | 150 | 150 |
|---|---|---|---|---|---|
| Polysorbate 80 (%, w/v) | | 0.02 | 0.02 | 0.02 | 0.02 |
| Trehalose (mM) | | 200 | 200 | 200 | 200 |
| | Turbidity | 0.2352 | 0.2135 | 0.2101 | 0.2110 |
| | RP-HPLC main peak (%) | 84.02 | 84.12 | 84.24 | 84.11 |
| | RP-HPLC pre-peak (%) | 4.10 | 4.03 | 3.97 | 4.10 |
| Day 0 at 5±3°C temperature | RP-HPLC post-peak (%) | 11.88 | 11.84 | 11.79 | 11.79 |
| | Heavy-chain Met262 oxidation rate (%) | 0.0 | 0.0 | 0.0 | 0.0 |
| | Light-chain Cys97 oxidation rate (%) | 2.3 | 1.9 | 2.0 | 2.1 |
| | Turbidity | 0.2105 | 0.2126 | 0.2107 | 0.2120 |
| | RP-HPLC main peak (%) | | | | |
| | RP-HPLC pre-peak (%) | | | | |
| 4 weeks at 5±3°C temperature | RP-HPLC post-peak (%) | | | | |
| | Heavy-chain Met262 oxidation rate (%) | 1.5 | 1.5 | 1.4 | 1.4 |
| | Light-chain Cys97 oxidation rate (%) | 1.9 | 1.4 | 1.4 | 1.5 |
| 2 weeks at 40±2°C temperature and 75±5% relative humidity | Turbidity | 0.3437 | 0.3167 | 0.2870 | 0.3098 |
| | RP-HPLC main peak (%) | | | | |
| | RP-HPLC pre-peak (%) | | | | |
| | RP-HPLC post-peak (%) | | | | |
| | Heavy-chain Met262 oxidation rate (%) | 0.7 | 0.5 | 0.4 | 0.3 |
| | Light-chain Cys97 oxidation rate (%) | 7.5 | 5.3 | 3.6 | 6.1 |
| | Turbidity | 0.4646 | 0.4010 | 0.3378 | 0.3807 |
| 4 weeks at 40±2°C temperature and 75±5% relative humidity | RP-HPLC main peak (%) | 66.84 | 68.89 | 70.62 | 69.52 |
| | RP-HPLC pre-peak (%) | 23.64 | 21.22 | 19.29 | 20.52 |
| | RP-HPLC post-peak (%) | 9.52 | 9.88 | 10.09 | 9.96 |
| | Heavy-chain Met262 oxidation rate (%) | 4.2 | 2.3 | 1.9 | 2.0 |
| | Light-chain Cys97 oxidation rate (%) | 9.8 | 7.2 | 4.6 | 6.9 |

It could be seen that, under the condition of 5±3°C temperature, Examples 1 to 3 all had a turbidity of 0.3 or less, an RP-HPLC main peak of 83% or more, an RP-HPLC pre-peak of 5% or less, an RP-HPLC post-peak of 13% or less, a methionine oxidation rate of 2% or less, and a cysteine oxidation rate of 3% or less, indicating that they were pharmaceutically acceptable stable examples. It could be seen that, under the conditions of 40±2°C temperature and 75±5% relative humidity, Examples 1 to 3 all had a turbidity of 0.6 or less, an RP-HPLC main peak of 63% or more, an RP-HPLC pre-peak of 30% or less, an RP-HPLC post-peak of 11% or less, a methionine oxidation rate of 5% or less, and a cysteine oxidation rate of 10% or less, indicating that they were pharmaceutically acceptable stable examples.

Through comparison between Examples 1 and 3, it was confirmed that, as the content of methionine increased, the turbidity was lower, the RP-HPLC pre-peak was lower, the RP-HPLC main peak was higher, the methionine oxidation rate was lower, and the cysteine oxidation rate was lower.

In particular, it was shown that the methionine oxidation rate was significantly lower in Example 3, in which the methionine concentration was 30 mM, than in the other Examples and the Comparative Examples, and that the oxidation rate decreased as the methionine concentration increased (FIG. 1).

Since it was confirmed in Experimental Example 2 that stability tended to improve as the methionine content increased, the experiment of Experimental Example 3 was conducted to further evaluate the stability when methionine is used at a concentration of 30 mM or higher.

### Experimental Example 3: Second Comparison of Antioxidant

The stability of Examples 4 to 7 and Comparative Example 2 prepared according to Experimental Example 1 was measured under the condition of 5±3°C temperature and the condition of 50°C temperature, and the results are shown in Table 3 below.

**[Table 3]**

| Evaluation Examples and results | | Example 4 | Example 5 | Example 6 | Example 7 | Comp. Example 2 |
|---|---|---|---|---|---|---|
| Antioxidant | | Not containing | Methionine 30 mM | Methionine 40 mM | Methionine 50 mM | Methionine 5 mM |
| Antibody (mg/ml) | | 150 | 150 | 150 | 150 | 150 |
| Polysorbate 80 (%, w/v) | | 0.02 | 0.02 | 0.02 | 0.02 | 0.02 |
| Trehalose (mM) | | 200 | 200 | 200 | 200 | 200 |
| | Turbidity | 0.2398 | 0.2092 | 0.2200 | 0.2221 | 0.2114 |
| | RP-HPLC main peak | 83.75 | 83.74 | 83.74 | 83.79 | 83.69 |
| 0 days at 5±3°C temperature | RP-HPLC pre-peak | 4.22 | 4.21 | 4.21 | 4.18 | 4.25 |
| | RP-HPLC post-peak | 10.80 | 12.06 | 12.05 | 12.02 | 12.06 |
| | SEC-HPLC monomer (%) | 97.06 | 97.16 | 96.94 | 96.84 | 97.00 |
| | SEC-HPLC high-molecular-weight component (%) | 1.85 | 1.78 | 1.82 | 1.85 | 1.84 |
| | SEC-HPLC low-molecular-weight component (%) | 1.08 | 1.06 | 1.24 | 1.29 | 1.15 |
| | Heavy-chain Met262 oxidation rate (%) | 1.1 | 0.8 | 1.1 | 1.1 | 1.1 |
| | Light-chain Cys97 oxidation rate (%) | 0.8 | 0.9 | 1.0 | 0.7 | 0.8 |
| | Turbidity | 0.2093 | 0.2030 | 0.2031 | 0.2041 | 0.21 |
| | RP-HPLC main peak | 83.64 | 83.73 | 83.66 | 83.63 | 83.68 |
| | RP-HPLC pre-peak | 4.21 | 4.21 | 4.22 | 4.23 | 4.21 |
| | RP-HPLC post-peak | 12.15 | 12.06 | 12.12 | 12.15 | 12.11 |
| | SEC-HPLC monomer (%) | 97.20 | 97.21 | 97.19 | 97.23 | 97.07 |
| **10** days at 5±3°C temperature | SEC-HPLC high-molecular-weight component (%) | 1.76 | 1.73 | 1.74 | 1.68 | 1.79 |
| | SEC-HPLC low-molecular-weight component (%) | 1.04 | 1.06 | 1.07 | 1.10 | 1.14 |
| | Heavy-chain Met262 oxidation rate (%) | 1.3 | 1.1 | 1.2 | 1.1 | 1.1 |
| | Light-chain Cys97 oxidation rate (%) | 0.7 | 0.9 | 0.8 | 0.7 | 0.7 |
| | Turbidity | 0.3965 | 0.3269 | 0.3299 | 0.3345 | 0.3548 |
| | RP-HPLC main peak | 69.92 | 71.76 | 71.69 | 71.64 | 71.4 |
| | RP-HPLC pre-peak | 20.11 | 17.99 | 18.08 | 17.98 | 18.51 |
| | RP-HPLC post-peak | 9.97 | 10.25 | 10.23 | 10.38 | 10.09 |
| | SEC-HPLC monomer (%) | 86.37 | 86.01 | 87.09 | 86.70 | 86.7 |
| 5 days at 50°C temperature | SEC-HPLC high-molecular-weight component (%) | 5.72 | 5.92 | 4.96 | 4.87 | 5.19 |
| | SEC-HPLC low-molecular-weight component (%) | 7.90 | 8.08 | 7.96 | 8.43 | 8.11 |
| | Heavy-chain Met262 oxidation rate (%) | 3.5 | 2.9 | 2.3 | 2.3 | 2.9 |
| | Light-chain Cys97 oxidation rate (%) | 3.2 | 1.2 | 1.1 | 1.2 | 1.5 |
| | Turbidity | 0.5848 | 0.4251 | 0.4246 | 0.4261 | 0.4709 |
| | RP-HPLC main peak | 62.04 | 65.15 | 65.06 | 65.05 | 64.22 |
| | RP-HPLC pre-peak | 29.26 | 25.65 | 25.64 | 25.76 | 26.77 |
| | RP-HPLC post-peak | 8.71 | 9.21 | 9.30 | 9.19 | 9.01 |
| 10 days at 50°C temperature | SEC-HPLC monomer (%) | 81.19 | 82.45 | 82.07 | 82.06 | 81.77 |
| | SEC-HPLC high-molecular-weight component (%) | 8.46 | 6.81 | 6.78 | 6.66 | 7.5 |
| | SEC-HPLC low-molecular-weight component (%) | 10.35 | 10.74 | 11.14 | 11.28 | 10.73 |
| | Heavy-chain Met262 oxidation rate (%) | 5.3 | 1.4 | 1.4 | 1.5 | 2.0 |
| | Light-chain Cys97 oxidation rate (%) | 6.0 | 3.6 | 3.9 | 3.9 | 4.6 |

It could be seen that, under the condition of 5±3°C temperature, Examples 4 to 7 had a turbidity of 0.3 or less, an RP-HPLC main peak of 83% or more, an RP-HPLC pre-peak of 5% or less, an RP-HPLC post-peak of 13% or less, an SEC-HPLC monomer of 94% or more, an SEC-HPLC high-molecular-weight component of 3% or less, an SEC-HPLC low-molecular-weight component of 3% or less, a methionine oxidation rate of 2% or less, and a cysteine oxidation rate of 3% or less, indicating that they were pharmaceutically acceptable stable examples. It could be seen that, under the condition of 50°C temperature, Examples 4 to 7 all had a turbidity of 0.6 or less, an RP-HPLC main peak of 55% or more, an RP-HPLC pre-peak of 37% or less, an RP-HPLC post-peak of 11% or less, an SEC-HPLC monomer of 77% or more, an SEC-HPLC high-molecular-weight component of 9% or less, an SEC-HPLC low-molecular-weight component of 16% or less, a methionine oxidation rate of 7% or less, and a cysteine oxidation rate of 6% or less, indicating that they were pharmaceutically acceptable stable examples.

Through comparison between Examples 4 to 7, it was shown that the turbidity tended to decrease as the methionine content increased, but there was no significant difference in the turbidity depending on the methionine content among the methionine-containing formulations of Examples 5 to 7.

As the methionine content increased, the RP-HPLC pre-peak was higher, but the RP-HPLC main peak was highest in Example 5 in which the methionine concentration was 30 mM.

As the methionine content increased, the SEC-HPLC high-molecular-weight component was lower, but the SEC-HPLC low-molecular-weight component was higher, and accordingly, the SEC-HPLC monomer was generally higher in Example 5 in which the methionine concentration was 30 mM.

Through comparison between Examples 4 to 7, it was shown that the methionine oxidation rate of the methionine-containing formulations was low, and there was no significant difference in the methionine oxidation rate depending on the methionine content among the methionine-containing formulations of Examples 5 to 7.

Through comparison between Examples 4 to 7, it was shown that the cysteine oxidation rate of the methionine-containing formulations was low, and there was no significant difference in the cysteine oxidation rate depending on the methionine content among the methionine-containing formulations of Examples 5 to 7. Example 5, in which the methionine concentration was 30 mM, showed the best stability in terms of the RP-HPLC main peak and the SEC-HPLC low-molecular-weight component and monomer, and also showed stability at a level similar to that of Examples 6 to 7, in which the methionine content was higher, in terms of the turbidity, the methionine oxidation rate, and the cysteine oxidation rate.

In order to evaluate the stability depending on the filling gas that may affect the oxidation rate of methionine and cysteine, in addition to the antioxidant, the experiment of Experimental Example 4 was conducted.

### Experimental Example 4: Comparison of Filling Gases

The stability of Examples 8 to 10 prepared according to Experimental Example 1 was measured under the conditions of 5±3°C temperature, the conditions of 40±2°C temperature and 75±5% relative humidity, the conditions of 1,000 lux light illumination, 25±2°C temperature, and 60±5% relative humidity, and the condition of agitation at 3,000 RPM, and the results are shown in Table 4 below.

**[Table 4]**

| Evaluation Examples and results | | Example 8 | Example 9 | Example 10 |
|---|---|---|---|---|
| Antioxidant | | Methionine 30 mM | Methionine 30 mM | Methionine 30 mM |
| Antibody (mg/ml) | | 150 | 150 | 150 |
| Polysorbate 80 (%, w/v) | | 0.02 | 0.02 | 0.02 |
| Trehalose (mM) | | 170 | 170 | 170 |
| Container | | Vial | Vial | Vial |
| Filling gas | | Air | Nitrogen | Oxygen |
| | Turbidity | | | |
| | RP-HPLC main peak | 84.86 | 84.96 | 84.90 |
| | RP-HPLC pre-peak | 3.40 | 3.37 | 3.42 |
| | RP-HPLC post-peak | 11.74 | 11.69 | 11.67 |
| | SEC-HPLC monomer (%) | 96.95 | 96.86 | 96.85 |
| 0 days at 5±3°C temperature | SEC-HPLC high-molecular-weight component (%) | 1.93 | 2.06 | 1.92 |
| | SEC-HPLC low-molecular-weight component (%) | 1.11 | 1.08 | 1.22 |
| | Heavy-chain Met262 oxidation rate (%) | 1.5 | 1.5 | 18.1 |
| | Light-chain Cys97 oxidation rate (%) | 0.4 | 0.4 | 0.5 |
| 4 weeks at 5±3°C temperature | Turbidity | 0.2436 | 0.2132 | 0.3377 |
| | RP-HPLC main peak | 84.50 | 84.66 | 83.93 |
| | RP-HPLC pre-peak | 3.73 | 3.52 | 4.43 |
| | RP-HPLC post-peak | 11.77 | 11.81 | 11.64 |
| | SEC-HPLC monomer (%) | 94.22 | 94.51 | 94.04 |
| | SEC-HPLC high-molecular-weight component (%) | 2.86 | 2.75 | 2.99 |
| | SEC-HPLC low-molecular-weight component (%) | 2.92 | 2.73 | 2.97 |
| | Heavy-chain Met262 oxidation rate (%) | 1.4 | 1.4 | 1.4 |
| | Light-chain Cys97 oxidation rate (%) | 0.8 | 0.5 | 1.1 |
| | Turbidity | 0.1891 | 0.1907 | 0.2283 |
| | RP-HPLC main peak | 73.85 | 76.09 | 71.47 |
| | RP-HPLC pre-peak | 15.48 | 12.89 | 18.34 |
| | RP-HPLC post-peak | 10.67 | 11.01 | 10.19 |
| 2 weeks at 40±2°C temperature and 75±5% relative humidity | SEC-HPLC monomer (%) | 90.79 | 91.03 | 90.05 |
| | SEC-HPLC high-molecular-weight component (%) | 3.85 | 3.78 | 4.21 |
| | SEC-HPLC low-molecular-weight component (%) | 5.35 | 5.19 | 5.74 |
| | Heavy-chain Met262 oxidation rate (%) | 1.5 | 1.5 | 1.7 |
| | Light-chain Cys97 oxidation rate (%) | 1.2 | 0.5 | 3.9 |
| | Turbidity | 0.3093 | 0.2802 | 0.4893 |
| 4 weeks at 40±2°C temperature and 75±5% relative humidity | RP-HPLC main peak | 70.11 | 73.75 | 64.12 |
| | RP-HPLC pre-peak | 19.56 | 15.38 | 26.57 |
| | RP-HPLC post-peak | 10.33 | 10.88 | 9.31 |
| | SEC-HPLC monomer (%) | 86.75 | 86.92 | 85.40 |
| | SEC-HPLC high-molecular-weight component (%) | 4.30 | 4.19 | 4.92 |
| | SEC-HPLC low-molecular-weight component (%) | 8.94 | 8.89 | 9.68 |
| | Heavy-chain Met262 oxidation rate (%) | 1.4 | 1.4 | 1.8 |
| | Light-chain Cys97 oxidation rate (%) | 2.0 | 0.6 | 5.5 |
| | Turbidity | 0.2100 | 0.2071 | 0.2464 |
| | RP-HPLC main peak | 83.75 | 84.36 | 82.90 |
| 72 hours at 1,000 lux light illumination, 25±2°C temperature and 60±5% relative humidity | RP-HPLC pre-peak | 4.85 | 3.89 | 6.01 |
| | RP-HPLC post-peak | 11.40 | 11.75 | 11.09 |
| | SEC-HPLC monomer (%) | 95.74 | 95.91 | 95.31 |
| | SEC-HPLC high-molecular-weight component (%) | 2.72 | 2.66 | 2.99 |
| | SEC-HPLC low-molecular-weight component (%) | 1.54 | 1.43 | 1.70 |
| | Heavy-chain Met262 oxidation rate (%) | 2.2 | 1.5 | 2.7 |
| | Light-chain Cys97 oxidation rate (%) | 1.1 | 0.4 | 1.6 |
| | Turbidity | 0.1805 | 0.1778 | 0.1920 |
| | RP-HPLC main peak | 84.67 | 84.73 | 84.49 |
| 4 hours under agitation at 3,000 RPM | RP-HPLC pre-peak | 3.48 | 3.34 | 3.71 |
| | RP-HPLC post-peak | 11.85 | 11.93 | 11.80 |
| | SEC-HPLC monomer (%) | 95.10 | 95.08 | 95.10 |
| | SEC-HPLC high-molecular-weight component (%) | 2.75 | 2.72 | 2.71 |
| | SEC-HPLC low-molecular-weight component (%) | 2.14 | 2.19 | 2.19 |
| | Heavy-chain Met262 oxidation rate (%) | 1.4 | 1.4 | 1.5 |
| | Light-chain Cys97 oxidation rate (%) | 0.7 | 0.6 | 0.9 |

It could be seen that, under the condition of 5±3°C temperature, Examples 8 to 10 all had a turbidity of 0.4 or less, an RP-HPLC main peak of 83% or more, an RP-HPLC pre-peak of 5% or less, an RP-HPLC post-peak of 13% or less, an SEC-HPLC monomer of 94% or more, an SEC-HPLC high-molecular-weight component of 3% or less, an SEC-HPLC low-molecular-weight of 3% or less, and a cysteine oxidation rate of 3% or less, indicating that they were pharmaceutically acceptable stable examples. However, Example 10 showed a methionine oxidation rate of 18% or more, which is a value indicating instability. It could be seen that, under the conditions of 40±2°C temperature and 75±5% relative humidity, Examples 8 to 10 had a turbidity of 0.6 or less, an RP-HPLC main peak of 63% or more, an RP-HPLC pre-peak of 30% or less, an RP-HPLC post-peak of 12% or less, an SEC-HPLC monomer of 85% or more, an SEC-HPLC high-molecular-weight component of 5% or less, an SEC-HPLC low-molecular-weight component of 10% or less, a methionine oxidation rate of 5% or less, and a cysteine oxidation rate of 10% or less, indicating that they were pharmaceutically acceptable stable examples.

It could be confirmed that, under the conditions of 1,000 lux light illumination, 25±2°C temperature and 60±5% relative humidity, Examples 8 to 10 all had a turbidity of 0.3 or less, an RP-HPLC main peak of 82% or more, an RP-HPLC pre-peak of 7% or less, an RP-HPLC post-peak of 12% or less, an SEC-HPLC monomer of 94% or more, an SEC-HPLC high-molecular-weight component of 3% or less, an SEC-HPLC low-molecular-weight component of 3% or less, a methionine oxidation rate of 3% or less, and a cysteine oxidation rate of 5% or less, indicating that they were pharmaceutically acceptable stable examples.

It could be confirmed that, under the condition of agitation at 3,000 RPM, Examples 8 to 10 all had a turbidity of 0.3 or less, an RP-HPLC main peak of 84% or more, an RP-HPLC pre-peak of 6% or less, an RP-HPLC post-peak of 12% or less, an SEC-HPLC monomer of 94% or more, an SEC-HPLC high-molecular-weight component of 3% or less, an SEC-HPLC low-molecular-weight component of 4% or less, a methionine oxidation rate of 2% or less, and a cysteine oxidation rate of 2% or less, indicating that they were pharmaceutically acceptable stable examples.

Example 9, in which the container was filled with nitrogen, showed the best stability in terms of the turbidity, the RP-HPLC main peak and pre-peak, the SEC-HPLC monomer, high-molecular-weight component and low-molecular-weight component, the methionine oxidation rate, and especially the cysteine oxidation rate.

Specifically, the 30 mM methionine-containing formulations prepared through the nitrogen filling process had an oxidation rate ≤ 0.7%, when measuring the light-chain Cys97 oxidation rate after storage for 4 weeks under the conditions of 40±2°C temperature and 75±5% relative humidity,

the 30 mM methionine-containing formulations prepared through the nitrogen filling had an oxidation rate ≤ 0.5%, when measuring the light-chain Cys97 oxidation rate after storage for 72 hours under the conditions of 1,000 lux light illumination, 25±2°C temperature, and 60±5% relative humidity, and

the 30 mM methionine-containing formulations prepared through the nitrogen filling had an oxidation rate ≤ 0.7%, when measuring the light-chain Cys97 oxidation rate after storage for 4 hours under the condition of agitation at 3,000 RPM (FIG. 2).

Example 10, in which the container was filled with oxygen, showed the worst results in terms of the turbidity, the RP-HPLC main peak and pre-peak, the SEC-HPLC monomer, high-molecular-weight component and low-molecular-weight component, the methionine oxidation rate, and the cysteine oxidation rate.

It was confirmed that Example 8, in which the container was filled with general air, was more stable than Example 10, but less stable than Example 9.

Overall, it was confirmed through the experiment of Experimental Example 4 that stability could be improved in various terms by filling the container with nitrogen. In addition, the experiment of Experimental Example 5 was conducted to evaluate the stability of the pharmaceutical formulation depending on a stopper insertion process during pre-filled syringe filling.

### Experimental Example 5. Comparison of Stopper Insertion Processes

The stability of Examples 11 to 13 prepared according to Experimental Example 1 was measured under the condition of 5±3°C temperature, the conditions of 40±2°C temperature and 75±5% relative humidity, and the condition of 50°C temperature, and the results are shown in Table 5 below.

**[Table 5]**

| Evaluation Examples and results | | Example 11 | Example 12 | Example 13 |
|---|---|---|---|---|
| Antioxidant | | Methionine 30 mM | Methionine 30 mM | Methionine 30 mM |
| Antibody (mg/ml) | | 150 | 150 | 150 |
| Polysorbate 80 (%, w/v) | | 0.02 | 0.02 | 0.02 |
| Trehalose (mM) | | 170 | 170 | 170 |
| Container | | Pre-filled syringe | Pre-filled syringe | Pre-filled syringe |
| Stopper insertion process | | Venting | Vacuum | Venting |
| Filling gas | | Air | Air | Nitrogen |
| | RP-HPLC main peak | 84.55 | 84.28 | 84.35 |
| | RP-HPLC pre-peak | 3.21 | 3.19 | 3.20 |
| | RP-HPLC post-peak | 12.35 | 12.53 | 12.45 |
| | SEC-HPLC monomer (%) | 96.36 | 96.50 | 96.42 |
| 0 days at 5±3°C temperature | SEC-HPLC high-molecular-weight component (%) | 2.00 | 2.00 | 2.00 |
| | SEC-HPLC low-molecular-weight component (%) | 1.65 | 1.50 | 1.58 |
| | Heavy-chain Met262 oxidation rate (%) | 1.5 | 1.5 | 1.5 |
| | Light-chain Cys97 oxidation rate (%) | 1.4 | 1.6 | 1.4 |
| | RP-HPLC main peak | 84.28 | 84.46 | 84.32 |
| | RP-HPLC pre-peak | 3.25 | 3.16 | 3.23 |
| | RP-HPLC post-peak | 12.47 | 12.32 | 12.45 |
| | SEC-HPLC monomer (%) | 96.24 | 95.91 | 96.20 |
| 3 weeks at 5±3°C temperature | SEC-HPLC high-molecular-weight component (%) | 2.16 | 2.60 | 2.15 |
| | SEC-HPLC low-molecular-weight component (%) | 1.60 | 1.49 | 1.64 |
| | Heavy-chain Met262 oxidation rate (%) | 1.8 | 1.8 | 1.6 |
| | Light-chain Cys97 oxidation rate (%) | 1.9 | 1.7 | 1.9 |
| 1 week at 40±2°C temperature and 75±5% relative humidity | RP-HPLC main peak | 83.31 | 83.29 | 83.63 |
| | RP-HPLC pre-peak | 5.36 | 5.37 | 4.95 |
| | RP-HPLC post-peak | 11.33 | 11.34 | 11.42 |
| | SEC-HPLC monomer (%) | 91.76 | 91.86 | 91.83 |
| | SEC-HPLC high-molecular-weight component (%) | 3.22 | 3.21 | 3.27 |
| | SEC-HPLC low-molecular-weight component (%) | 5.01 | 4.93 | 4.90 |
| | Heavy-chain Met262 oxidation rate (%) | 1.5 | 1.4 | 1.4 |
| | Light-chain Cys97 oxidation rate (%) | 3.2 | 3.0 | 2.3 |
| | RP-HPLC main peak | 75.20 | 75.14 | 76.45 |
| | RP-HPLC pre-peak | 13.96 | 14.03 | 12.44 |
| | RP-HPLC post-peak | 10.84 | 10.83 | 11.11 |
| 2 weeks at 40±2°C temperature and 75±5% relative humidity | SEC-HPLC monomer (%) | 89.71 | 89.38 | 89.85 |
| | SEC-HPLC high-molecular-weight component (%) | 3.63 | 3.86 | 3.49 |
| | SEC-HPLC low-molecular-weight component (%) | 6.66 | 6.76 | 6.66 |
| | Heavy-chain Met262 oxidation rate (%) | 1.6 | 1.6 | 1.5 |
| | Light-chain Cys97 oxidation rate (%) | 3.5 | 4.1 | 2.3 |
| | RP-HPLC main peak | 72.53 | 72.49 | 74.09 |
| 3 weeks at 40±2°C temperature and 75±5% relative humidity | RP-HPLC pre-peak | 17.00 | 17.07 | 15.27 |
| | RP-HPLC post-peak | 10.46 | 10.44 | 10.65 |
| | SEC-HPLC monomer (%) | 87.29 | 87.87 | 87.89 |
| | SEC-HPLC high-molecular-weight component (%) | 4.14 | 3.93 | 4.05 |
| | SEC-HPLC low-molecular-weight component (%) | 8.57 | 8.21 | 8.06 |
| | Heavy-chain Met262 oxidation rate (%) | 1.7 | 2.0 | 1.9 |
| | Light-chain Cys97 oxidation rate (%) | 4.4 | 3.9 | 2.5 |
| | RP-HPLC main peak | 68.83 | 69.08 | 70.36 |
| | RP-HPLC pre-peak | 21.40 | 21.20 | 19.56 |
| | RP-HPLC post-peak | 9.77 | 9.71 | 10.08 |
| | SEC-HPLC monomer (%) | 84.58 | 84.75 | 84.97 |
| 1 week at 50°C temperature | SEC-HPLC high-molecular-weight component (%) | 5.13 | 4.61 | 4.51 |
| | SEC-HPLC low-molecular-weight component (%) | 5.01 | 4.93 | 4.90 |
| | Heavy-chain Met262 oxidation rate (%) | 1.4 | 1.6 | 1.5 |
| | Light-chain Cys97 oxidation rate (%) | 3.8 | 3.9 | 2.2 |
| | RP-HPLC main peak | 61.59 | 61.53 | 64.16 |
| | RP-HPLC pre-peak | 29.78 | 29.83 | 26.66 |
| | RP-HPLC post-peak | 8.63 | 8.64 | 9.18 |
| | SEC-HPLC monomer (%) | 80.41 | 80.36 | 80.94 |
| 2 weeks at 50°C temperature | SEC-HPLC high-molecular-weight component (%) | 5.82 | 5.90 | 5.73 |
| | SEC-HPLC low-molecular-weight component (%) | 13.77 | 13.73 | 13.33 |
| | Heavy-chain Met262 oxidation rate (%) | 1.9 | 1.9 | 1.8 |
| | Light-chain Cys97 oxidation rate (%) | 6.1 | 5.5 | 2.0 |
| | RP-HPLC main peak | 55.55 | 56.00 | 58.74 |
| | RP-HPLC pre-peak | 36.55 | 35.82 | 32.58 |
| | RP-HPLC post-peak | 7.90 | 8.18 | 8.69 |
| | SEC-HPLC monomer (%) | 77.81 | 77.85 | 78.47 |
| 3 weeks at 50°C temperature | SEC-HPLC high-molecular-weight component (%) | 6.82 | 6.73 | 6.70 |
| | SEC-HPLC low-molecular-weight component (%) | 15.37 | 15.42 | 14.83 |
| | Heavy-chain Met262 oxidation rate (%) | 2.0 | 1.9 | 1.8 |
| | Light-chain Cys97 oxidation rate (%) | 6.6 | 5.6 | 2.6 |

It could be seen that, under the condition of 5±3°C temperature, Examples 11 to 13 all had an RP-HPLC main peak of 83% or more, an RP-HPLC pre-peak of 5% or less, an RP-HPLC post-peak of 13% or less, an SEC-HPLC monomer of 94% or more, an SEC-HPLC high-molecular-weight component of 3% or less, an SEC-HPLC low-molecular-weight component of 3% or less, a methionine oxidation rate of 2% or less, and a cysteine oxidation rate of 3% or less, indicating that they were pharmaceutically acceptable stable examples. It could be seen that, under the condition of 40±2°C temperature and 75±5% relative humidity, Examples 11 to 13 all had an RP-HPLC main peak of 63% or more, an RP-HPLC pre-peak of 30% or less, an RP-HPLC post-peak of 12% or less, an SEC-HPLC monomer of 85% or more, an SEC-HPLC high-molecular-weight component of 5% or less, an SEC-HPLC low-molecular-weight component of 10% or less, a methionine oxidation rate of 5% or less, and a cysteine oxidation rate of 10% or less, indicating that they were pharmaceutically acceptable stable examples.

It could be seen that, under the condition of 50°C temperature, Examples 11 to 13 all had an RP-HPLC main peak of 55% or more, an RP-HPLC pre-peak of 37% or less, an RP-HPLC post-peak of 11% or less, an SEC-HPLC monomer of 77% or more, an SEC-HPLC high-molecular-weight component of 7% or less, an SEC-HPLC low-molecular-weight component of 16% or less, a methionine oxidation rate of 3% or less, and a cysteine oxidation rate of 7% or less, indicating that they were pharmaceutically acceptable stable examples.

Example 13, in which the venting and nitrogen filling process was applied during insertion of the stopper into the pre-filled syringe, showed the highest stability in terms of the RP-HPLC main peak and pre-peak, the SEC-HPLC monomer, high-molecular-weight component and low-molecular-weight component, and the cysteine oxidation rate.

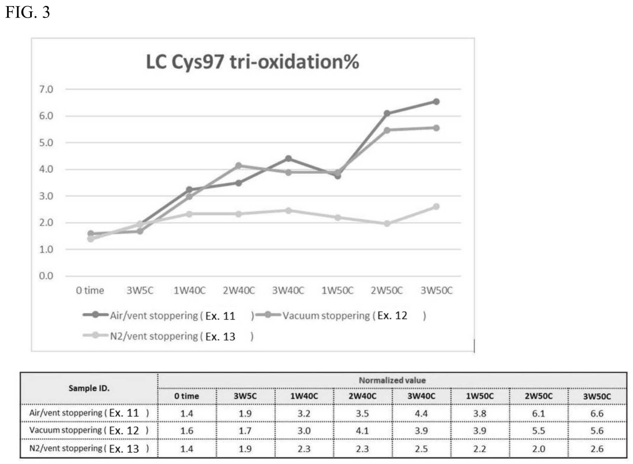

In particular, the cysteine oxidation rate significantly decreased when the venting and nitrogen filling process was applied (FIG. 3).

Example 12, in which the vacuum filling process was applied during insertion of the stopper into the pre-filled syringe, showed higher stability than Example 11, in which only the venting process was applied without nitrogen filling, in terms of the RP-HPLC main peak and pre-peak and the cysteine oxidation rate. Overall, it was confirmed that both the case in which the venting and nitrogen filling process was applied during insertion of the stopper into the pre-filled syringe and the case in which the vacuum process was applied showed higher stability than the case in which the venting process was applied without nitrogen filling, and that the stability was higher in the nitrogen filling process than in the vacuum process.

## Claims

1. A stable pharmaceutical formulation comprising:
(A) about 150 mg/ml secukinumab;
(B) about 30 to 50 mM methionine;
(C) about 0.001 to 1% (w/v) surfactant;
(D) about 170 to 200 mM stabilizer which is a sugar, a sugar alcohol, or a mixture thereof; and
(E) about 0.1 to 50 mM histidine buffer.

2. The stable pharmaceutical formulation of claim 1, wherein a concentration of the methionine is about 30 mM.

3. The stable pharmaceutical formulation of claim 1, wherein the surfactant is polysorbate 80.

4. The stable pharmaceutical formulation of claim 1, wherein the stabilizer is trehalose.

5. The stable pharmaceutical formulation of claim 1, wherein the stabilizer is 170 mM trehalose.

6. A stable pharmaceutical formulation comprising:
(A) about 150 mg/ml secukinumab;
(B) about 30 to 50 mM methionine;
(C) about 0.001 to 1% (w/v) surfactant;
(D) about 170 to 200 mM stabilizer which is a sugar, a sugar alcohol, or a mixture thereof; and
(E) about 0.1 to 50 mM histidine buffer,
the formulation being prepared through a process of filling a container with nitrogen during the manufacturing of a drug product.

7. The stable pharmaceutical formulation of claim 6, wherein a concentration of the methionine is about 30 mM.

8. The stable pharmaceutical formulation of claim 6, wherein the surfactant is polysorbate 80.

9. The stable pharmaceutical formulation of claim 6, wherein the stabilizer is 170 mM trehalose.

10. The stable pharmaceutical formulation of any one of claims 6 to 9, wherein the formulation has:
a. an oxidation rate ≤ 0.7% for a 30 mM methionine-containing formulation prepared through a nitrogen filling process, when measuring an oxidation rate of light-chain Cys97 after 4 weeks of storage under conditions of 40±2°C temperature and 75±5% relative humidity; and/or
b. an oxidation rate ≤ 0.5% for a 30 mM methionine-containing formulation prepared through a nitrogen filling process, when measuring an oxidation rate of light-chain Cys97 after 72 hours of storage under conditions of 1,000 lux light illumination, 25±2°C temperature, and 60±5% relative humidity; and/or
c. an oxidation rate ≤ 0.7% for a 30 mM methionine-containing formulation prepared through a nitrogen filling process, when measuring an oxidation rate of light-chain Cys97 after 4 hours of storage under conditions of agitation at 3,000 RPM.

11. A stable pharmaceutical formulation comprising:
(A) about 150 mg/ml secukinumab;
(B) about 30 to 50 mM methionine;
(C) about 0.001 to 1% (w/v) surfactant;
(D) about 170 to 200 mM stabilizer which is a sugar, a sugar alcohol, or a mixture thereof; and
(E) about 0.1 to 50 mM histidine buffer,
the formulation being prepared through a process of filling a container with nitrogen during the manufacturing of a drug product and a venting and nitrogen filling process during insertion of a stopper into a pre-filled syringe.

12. The stable pharmaceutical formulation of claim 11, wherein a concentration of the methionine is about 30 mM.

13. The stable pharmaceutical formulation of claim 11, wherein the surfactant is polysorbate 80.

14. The stable pharmaceutical formulation of claim 11, wherein the stabilizer is 170 mM trehalose.
